Europäisches Patentamt

European Patent Office

Office européen des brevets

(11) Publication number: **0 181 170 B1**

# EUROPEAN PATENT SPECIFICATION

(45) Date of publication of patent specification: **04.03.92** (51) Int. Cl.⁵: **A61K 35/74**

(21) Application number: **85307945.7**

(22) Date of filing: **01.11.85**

(54) **Hypocholesterolemically active products.**

(30) Priority: **05.11.84 JP 231266/84**

(43) Date of publication of application:
**14.05.86 Bulletin 86/20**

(45) Publication of the grant of the patent:
**04.03.92 Bulletin 92/10**

(84) Designated Contracting States:
**CH DE FR GB IT LI NL SE**

(56) References cited:
DE-A- 2 134 179      DE-A- 2 421 084
FR-A- 2 411 008      FR-M- 3 614
FR-M- 5 601          GB-A- 930 107

W. WATANUKI et al.: "Influence of gastrointestinal flora on the lipid metabolism of the host rats", & CHONAI FURORA TO EIYA RIKEN CHONAI FURORA SHINPOJUMU; 3rd 1982 (Pub. 1983), 107-20

JOURNAL OF DAIRY SCIENCE, vol. 62, no. 10, 1979, pages 1685-1694 K.H.SHAHANI et al.: "Nutritional and heelthful aspects of cultured and culture-containing dairy foods"

(73) Proprietor: **KABUSHIKI KAISYA ADVANCE**
**5-7, Nihonbashi Kobuna-cho**
**Chuo-ku Tokyo 103(JP)**

(72) Inventor: **Kawai, Yasuo**
**2-8-12, Mouridai**
**Atsugi-shi Kanagawa(JP)**
Inventor: **Yazawa, Kazunaga**
**Green-Haitsu D1-501 571, Unomori**
**Sagamihara-shi Kanagawa(JP)**
Inventor: **Shimohashi, Hirotaka**
**1-877, Ogawa-cho**
**Kodaira-shi Tokyo(JP)**

(74) Representative: **Harrison, David Christopher**
**et al**
**MEWBURN ELLIS & CO 2/3 Cursitor Street**
**London EC4A 1BO(GB)**

EP 0 181 170 B1

BIOLOGICAL ABSTRACTS, vol. 59, 1975, abstract no. 65408 Philadelphia, Pa.US; G.E.MOTT et al.: "Lowering of serum cholesterol by intestinal bacteria in cholesterol-fed piglets"

BIOLOGICAL ABSTRACTS, vol. 75, 1983, abstract no. 89936 Philadelphia, Pa. US; K.K. GRUNEWALD: "Serum cholesterol levels in rats fed skim milk fermented by Lactobacillus acidophilus"

CHEMICAL ABSTRACTS, volume 102, no. 17, 29th April 1985, page 497, abstract no. 147984g Columbus, Ohio, US

## Description

The present invention relates to hypocholesterolemic active products, processes for producing the same, pharmaceutical preparations containing the same, and a novel strain of the genus Bifidobacterium suitable for use in the production of the same.

As is well-known in the art, pharmaceutical preparations such as clofibrate-containing preparations have been proposed as a therapeutical or preventive medicine suitable for atherosclerosis or hyperlipidemia, which is one of a typical middle-aged or geriatric disease. However, the desired purposes are not fully satisfied by these known medicines from the viewpoint of, for example, pharmacological effects and side-effects, and therefore, there is a need to develop more effective medicines.

Furthermore, FR-A-2411008 discloses that certain strains of B. adolescentis, stabilized by lactulose may be effective for encouraging Bifidobacterial flora in the intestine.

An object of the present invention is to provide novel bacterial cell products capable of effectively reducing the serum cholesterol level in mammals, and to provide processes for preparing a bacterial cell products capable of effectively reducing the serum cholesterol level in mammals, pharmaceutical preparations containing the bacterial cell products.

A still further object of the present invention is to provide a novel strain of Bifidobacterium expressing a product having hypocholesterolemic activity in mammals.

In accordance with the present invention, there is provided the use of dead cells, living cells, or mixtures thereof of microorganisms belonging to the genus Bifidobacterium having hypocholesterolemic activity in mammals, for the preparation of a pharmaceutical composition for controlling the serum cholesterol level of patients. Such microorganisms can be found amongst the group consisting of B. adolescentis, B. breve and B. longum.

In accordance with the present invention, there is further provided a novel strain of B. adolescentis AD0050 (FERM BP-922).

The products and compositions are used for lowering the serum cholesterol level of mammals by orally administering them to the mammals.

The present inventors have found that various living cells and dead cells of microorganisms belonging to the genus Bifidobacterium can effectively reduce the serum cholesterol level in mammals. These microorganisms derived from gastrointestinal bacteria are substantially non-toxic when orally administered, and have an extremely high pharmacological activity.

The types and morphological characteristics, the screening methods, the preparation methods of strains, and the pharmacological effects of the microorganisms according to the present invention will now be explained in detail hereinbelow.

### Microorganisms

Microorganisms suitable for use in the preparation of the bacterial cell product according to the present invention are those belonging to the genus Bifidobacterium, especially Bifidobacterium adolescentis, B. breve, B. longum, B. infantis, B. bifidum, B. thermophilum, B. pseudolongum, B. coryneforme, B. asteroides, B. indicum and B. suis. A typical example of such microorganisms is B. adolescentis AD0050 which has been deposited since September 11, 1984 in the Fermentation Research Institute (FRI) in Japan under the number FERM P-7842 (all the numbers quoted as "FERM-P" refer to the deposition numbers of said Institute) and transferred to the Fermentation Research Institute (FRI) (i.e., International Depository Authority under the Budapest Treaty in Japan) as FERM BP-922.

### General Microbiological Characteristics

The general microbiological characteristics of the microorganisms in the present invention are the same as those of known microorganisms belonging to the same class. That is, the general microbiological characteristics, cultivation methods, and other properties correspond to those described in the following articles:

1) Bergey's Manual of Determinative Bacteriology, 8th ed., 490-509 (1974)

2) Poupard, J.A., Husain, I. and Norris, R.F. (1973): Bacteriol. Rev. 37, 136-165

3) Mituoka, T. (1969), Jpn. J. Bacteriol. 24, 261-280

4) Shimohashi, H., and Mutai, M. (1977). J. Gen. Microbiol.

The typical microbiological characteristics of the above-exemplified strains according to the present invention are summarized in Table 1.

Table 1

| Characteristics of B. adolescentis AD0050 | |
|---|---|
| Gram strain | + |
| Gas production from glucose | − |
| Growth at below 15°C and at 45°C | − |
| Catalase | − |
| Fermentation of | |
| Arabinose | + |
| Xylose | + |
| Ribose | + |
| Mannose | +S/+ [*1] |
| Fructose | + |
| Sucrose | + |
| Maltose | + |
| Lactose | + |
| Trehalose | V [*4] |
| Melibiose | + |
| Raffinose | + |
| Melezitose | −/+S [*2] |
| Dextrin | V [*4] |
| Starch | V [*4] |
| Glycogen | V [*4] |
| Inulin | V [*4] |
| Mannitol | V [*4] |
| Sorbitol | V [*4] |
| Inositol | − |
| Esculin | + |
| Amygdalin | + |
| Cellobiose | +S [*3] |
| Salicin | + |
| α−methyl glucoside | + |

[*1] Slowly reacted, rarely negative

[*2] Negative, rarely reacted as positive at later stage

[*3] S: Slowly reacted

[*4] V: Variable

The cultivation of these microorganisms is conventional as mentioned above. For example, the bacterial cells of Bifidobacterium can be cultivated in a GAM broth medium under an anaerobical condition, and can be harvested by centrifugation of the culture.

## Composition of GAM broth medium

"Nissui Seiyaku K.K." code 05422

|  |  |
|---|---|
|  | 59.0 g |
| Peptone | 10.0 g |
| Soybean peptone | 3.0 g |
| Proteose peptone | 10.0 g |
| Digested serum powder | 13.0 g |
| Yeast extract | 5.0 g |
| Meat extract | 2.2 g |
| Liver extract powder | 1.2 g |
| Glucose | 3.0 g |
| KH2PO4 | 2.5 g |
| NaCl | 3.0 g |
| Soluble starch | 5.0 g |
| L-cysteine hydrochloride | 0.3 g |
| Sodium thioglycolate | 0.3 g |
| Distilled water | to 1 litter |

(pH 7.3 $\pm$ 0.1)

The resultant cells can be directly used as a pharmaceutical agent in the form of living cells or dead cells obtained by, for example, heat-treatment, or in the form of the cells destroyed by, for example, ultrasonic treatment. The term "dead cells" used herein means the entire portions or partial portions of the above-mentioned destroyed cells.

The dead cells can be also used as a starting material for fractionating, extracting, and purifying active elements contained therein. This is because the desired pharmacological activities (i.e., hypocholesterolemic activity in mammals) are based on substance components contained in the cells, and because not only the living cells but also the dead cells have these pharmacological activities.

Screening Methods

The screening of the microorganisms can be carried out by a method of Mituoka, T., (1971), J. Jap. Assoc. Infect. Dis. 45, 406.

As described in this literature, faeces obtained from healthy adults were diluted 10 fold in the following diluent and were smeared on MPN agar (Tanaka, R. et al. (1980) Appl. Environ. Microbiol. 40, 866-869; the composition of the broth medium is as shown below) for Bifidobacterium and were cultivated at 37°C for 48 to 120 hours. The formed colonies were counted and were randomly isolated. The colony type and catalase activity, and the Gram strain and shape of cell were determined and the isolates were identified and classified by examining their physiological, biochemical, and serological properties.

| Composition of diluent | |
|---|---|
| KH2PO4 | 4.5 g |
| Na2HPO4 | 6.0 g |
| L-cysteine hydrochloride | 0.5 g |
| Tween 80[*1] | 0.5 g |
| Agar | 1.0 g |
| Distilled water | 1000 ml |

*1: Polyoxyethylene (20) sorbitan monooleate available from Atlas Powder Co.

## Composition of MPN agar

| | |
|---|---|
| Lactose | 2 g |
| (NH4)2SO4 | 0.5 g |
| K2HOP4 | 0.1 g |
| MgSO4.7H2O | 10 g |
| FeSO4.7H2O | 0.5 g |
| MnSO4.2H2O | 0.4 g     0.5 ml |
| NaCl | 0.5 g |
| Distilled water | 250 ml |
| 0.1% Resazurin | 0.1 ml |
| Biotin | 0.01 mg |
| Pantothenic acid | 0.2 mg |
| Riboflavin | 0.1 mg |
| Adenine | 0.1 mg |
| Guanine | 0.1 mg |
| Xanthine | 0.1 mg |
| Uracil | 0.1 mg |
| Tween 80[*1] | 0.1 g |
| 10% Pyruvic acid | 0.1 ml |
| 8% Na2CO3 | 5.0 ml |
| 3% L-cysteine hydrochloride | 1 ml |
| Nalidixic acid | 10 mg |
| 1.6% Bromcresol purple | 0.1 ml |
| Agar | 2 g |

Distilled water                    to 100 ml

pH=6.8

*1:  Polyoxyethylene (20) sorbitan monooleate available from Atlas Powder Co.

Preparation of Bacterial Cells

The living cells and dead cells of the microorganisms used in the present invention, suitable for use as a hypocholesterolemic agent, are typically prepared as follows:

1. Preparation Example of the Living Cells

Each strain of the above-mentioned microorganisms is inoculated into 5 liters of the above-mentioned GAM broth medium (in the case of Bifidobacterium) and is stationarily cultivated under anaerobic conditions, at 37°C for 8 hours to yield the subsequent culture broth containing $10^9$/ml of the living cells. The microorganisms are harvested by continuous centrifugation at 12,000 rpm. The bacterial cells are washed with physiological saline, and are then suspended in physiological saline to obtain 50 ml of the cell suspension containing $10^{11}$ cells/ml.

2. Preparation Example of the Dead Cells

The living cells obtained as mentioned in the above Example are further washed twice with physiological saline (0.85% NaCl solution) and are then suspended in the same solution. Fifty ml of the cell suspension was thus obtained, and was heated at 115°C for 10 minutes to form the desired cell suspension containing the dead cells.

Pharmacological Actions

1. Pharmacological Effects

a) As shown in each example hereinbelow, the bacterial cell products obtained from the above-mentioned microorganisms of the present invention can make an extremely effective reduction of a serum cholesterol level in mammals. Accordingly, the bacterial cell products embodying the present invention are useful as a therapeutical or preventive medicine for diseases closely related to atherosclerosis (comprising cerebral atherosclerosis, cerebral thrombus, and coronary atherosclerosis), hyperlipidemia, hyperlipoproteinemia, xanthomatosis, cholecystolithiasis, hypertension, diabetes, cardiopathia and nephropathy (nephrosis syndrome).

The term "hypocholesterolemic activity" used herein means the activity of reducing a serum cholesterol level by about 20% or more as compared with a control in mammals. This is because the reducing rate of about 20% or more is a remarkable pharmacological effect, since standard deviations in these animal tests are deemed to be generally ±10%.

b) Pharmaceutical compositions embodying to the present invention can be generally applied in a dosage of $10^7$ to $10^{14}$ cells/kg body weight, more desirably $10^9$ to $10^{12}$ cells/kg body weight, by, for example, an oral administration. The pharmaceutical compositions can be in the form of, for example, suspensions in physiological saline solutions, powder, granules, tablets, and capsules. They can be optionally prepared by using conventional appropriate carriers, bulk fillers and diluents.

2. Acute Toxicity

As shown in the examples hereinbelow, and LD50 of the living cells embodying the present invention is $4.9 \times 10^9$ to $8.3 \times 10^9$ cells/mouse (intraperitoneal administration) and that of the dead cells is more than $6 \times 10^{10}$ cells/mouse (intraperitoneal administration).

Both the living and dead cells are substantially nontoxic on oral administration.

EXAMPLES

The present invention will now be further illustrated by, the following examples.

Example 1

B. adolescentis AD0050 (FERM BP-922) and B. longum AD0051 were inoculated into 5 liters of the GAM broth medium. It was then stationarily cultivated under anaerobic conditions, at 37°C for 5 hours to yield a culture broth containing $10^9$/ml of the living cells. The microorganisms were harvested by continuous

7

centrifugation at 12,000 rpm. The bacterial cells were washed with physiological saline and were then suspended in physiological saline to obtain 50 ml of the cell suspension containing $10^{11}$ cells/ml as the living cell sample.

To obtain a dead cell sample, the above-mentioned living cell sample was further washed twice with physiological saline (0.85% NaCl solution) and was then suspended in the same solution. Fifty ml of the cell suspension was thus obtained and heated at 115°C for 10 minutes to form the desired cell suspension containing $10^{11}$ cells/ml. These samples were lyophilized and orally administered at a dosage corresponding to $10^{11}$ cells/day to GBH-9, HF, and conventional rats (Fischer 344 rats, 13-16 week-old males, average body weight of 240 g, 10 males in each group) for 8 weeks.

GBH-9 rats: gnotobiotic rats inoculated with Bacteroides, Eubacterium, Fusobacterium, Propionibacterium, Peptostreptococcus, Clostridium, Streptococcus, Staphylococcus and E. coli obtained from human faeces

HF rats: gnotobiotic rats inoculated with bacteria from the faeces of a healthy human

Arterial blood was then collected from the abdominal aorta of these rats and the serum samples were separated by centrifugation from the whole blood.

The cholesterol level was determined by using Choleskit (manufactured by Kanto Kagaku K.K., Zurkowski method).

Moreover, determinations similar to these mentioned above were made with newborn rats (21 day-old) and conventionalized rats (ex-germfree rats housed for five weeks under conventional conditions).

The results are shown in Table 2 (living cells) and Table 3 (dead cells).

"Reduction rates(%)" listed in these tables are the reduction rates to the serum cholesterol levels of the control groups which are not dosed with the sample. The composition (% by weight) of the diet is shown in Table 4.

## Table 2

| Rats | Administered bacteria | Serum cholesterol (mg/dl) | Reduction rate (%) |
|---|---|---|---|
| GBH-9 | B. longum | 38.7 ± 2.2 | 44.5 |
|  | B. adolescentis | 36.7 ± 4.0 | 47.3 |
|  | Control (nothing administered) | 69.7 ± 9.0 |  |
| HF | B. adolescentis | 45.4 ± 8.7 | 35.7 |
|  | Control (nothing administered) | 70.7 ± 7.0 |  |
| Conventionalized (loaded with 0.5% CHL[*1]) | B. adolescentis | 71.1 ± 13.1 | 46.6 |
|  | Control (nothing administered) | 133.1 ± 13.1 |  |
| Newborn | B. adolescentis | 89.8 ± 4.9 | 31.9 |
|  | Control (nothing administered) | 131.9 ± 10.3 |  |

*1 CHL: cholesterol

## Table 3

| Rats | Administered bacteria | Serum cholesterol (mg/dl) | Reduction rate (%) |
|------|----------------------|---------------------------|--------------------|
| GBH-9 | B. longum | 44.1 ± 2.2 | 36.7 |
|  | B. adolescentis | 43.4 ± 4.0 | 37.8 |
|  | Control (nothing administered) | 69.7 ± 9.0 |  |
| HF | B. adolescentis | 49.7 ± 8.7 | 29.7 |
|  | Control (nothing administered) | 70.7 ± 7.0 |  |
| Conventionalized (loaded with 0.5% CHL[*1]) | B. adolescentis | 81.7 ± 2.7 | 38.6 |
|  | Control (nothing administered) | 133.1 ± 13.1 |  |
| Newborn | B. adolescentis | 90.8 ± 4.9 | 31.2 |
|  | Control (nothing administered) | 131.9 ± 10.3 |  |

*1 CHL: cholesterol

Table 4

| Composition | wt.% |
|-------------|------|
| Casein | 20 |
| Soybean oil | 10 |
| Wheat starch | 61 |
| Minerals | 4 |
| Vitamin mixture | 2 |
| Powdered filter paper | 3 |

Example 2

Living cells of bacteria prepared according to the above-mentioned living cell preparation method were intraperitoneally administered to ICR mice (6 week-old males, average body weight of 30.0 ± 0.7 g) with 0.5 mg of a bacterial suspension containing $9 \times 10^9$, $9 \times 10^8$ and $9 \times 10^7$ cells per mouse (10 mice in each group). Thanatobiologic observation of the mice was carried out for 14 days.

The LD50 values (viable cell number/mouse) calculated according to a Behrens-Kärber method are shown in Table 5.

In the case of the dead cells of the above-mentioned strains the LD50 values corresponded to more than $6 \times 10^{10}$ cells/mouse (intraperitoneal dosage). Both the living and dead cells of the above-mentioned strains were nontoxic in oral administration.

Table 5

| Strains | LD50 (viable cell number/mouse) |
|---|---|
| B. adolescentis | $5.5 \times 10^9$ |
| B. longum | $4.9 \times 10^9$ |

Example 3 (Pharmaceutical preparations)

1. A 50 mg amount (corresponding to $5 \times 10^{10}$ cells) of freeze dried powder of B. adolscentis AD0050 living cells or dead cells prepared according to the above-mentioned cell preparation method was uniformly mixed with 950 mg of purified starch powder and then formed into tablets for oral administration. Each tablet corresponds to a dosage of $10^9$ cells/kg body weight for a human adult having a body weight of 50 kg.

A tablet obtained from 500 mg of the above-mentioned freeze dried powder by mixing with 500 mg of purified starch powder corresponds to a dosage of $10^{10}$ cells/kg body weight.

Thus, the cell products can be converted into the desired dosage form having a predetermined activity by mixing with pharmaceutically acceptable carriers based on the above-mentioned standard dosage.

**Claims**

1. The use of living cells, dead cells, or a mixture thereof of microorganisms belonging to the genus Bifidobacterium and having hypocholesterolemic activity in mammals for the preparation of a pharmaceutical composition for controlling the serum cholesterol level of patients.

2. The use according to claim 1, in which said microorganism is at least one member selected from B. adolescentis, B. breve and B. longum.

3. The use according to claim 1 or claim 2, in which said microorganism is B. adolescentis FERM BP-922.

4. A pharmaceutical composition for controlling the serum cholesterol level of patients comprising a preventively or therapeutically effective amount of living cells, dead cells, or a mixture thereof of Bifidobacterium adolescentis FERM BP-922 and a pharmaceutically acceptable carrier therefor.

**Revendications**

1. Utilisation de cellules vivantes, de cellules mortes ou d'un mélange de cellules vivantes et mortes de micro-organismes appartenant au genre *Bifidobacterium* et ayant une activité hypocholestérolémique chez les mammifères, pour la préparation d'une composition pharmaceutique propre à contrôler le taux sérique de chlolestérol de patients.

2. Utilisation selon la revendication 1, dans laquelle ledit micro-organisme est au moins un membre choisi parmi *B. adolescentis*, *B. breve* et *B. longum.*

3. Utilisation selon la revendication 1 ou 2, dans laquelle ledit micro-organisme est *B. adolescentis* FERM BP-922.

4. Composition pharmaceutique propre à contrôler le taux sérique de cholestérol de patients, comprenant une quantité préventivement ou thérapeutiquement efficace de cellules vivantes, de cellules mortes ou d'un mélange de cellules vivantes et mortes de *Bifidobacterium adolescentis* FERM BP-922 et un excipient acceptable du point de vue pharmaceutique pour ces cellules.

**Patentansprüche**

1. Verwendung von lebenden oder toten Zellen oder einer Mischung aus lebenden und toten Zellen von Mikroorganismen aus der Gattung der Bifidobakterien, die eine hypercholesterolemische Aktivität in Säugetieren entwickeln, zur Herstellung eines pharmazeutischen Mittels für die Steuerung des Cholesterinspiegels im Serum eines Patienten.

2. Verwendung des Mittels nach Anspruch 1, in dem dieser Mikroorganismus mindestens eine Verbindung aus der Gruppe der B. adolescentis, B. breve und B. longum enthält.

3. Verwendung des Mittels nach einem der Ansprüche 1 oder 2, in dem der Mikroorganismus B. adolescentis FERM BP-922 ist.

4. Ein pharmazeutisches Mittel für die Steuerung des Cholesterinspiegels im Serum eines Patienten, das eine präventiv oder therapeutisch wirksame Menge von lebenden Zellen, toten Zellen oder eine Mischung aus solchen Zellen aus dem Bifidobacterium adolescentis FERM BP-922 und einen geeigneten pharmazeutischen Träger aufweist.